# EUROPEAN PATENT APPLICATION

(11) **EP 1 462 804 A1**
(43) Date of publication of application: **29.09.2004**
(21) Application number: 03075881.7
(22) Date of filing: 25.03.2003
(51) Int. Cl.: G01N 33/68, C12Q 1/70

(54) **Method for the detection of a pathogenic form of a prion protein**

(71) Applicant: Stichting Sanquin Bloedvoorziening, 1066 CX Amsterdam (NL)
(72) Inventor: van Oers, Josephus Wilhelmus Alphonsus Maria, 1506 TB Zaandam (NL); van der Vorst, Teun Jan Karel, 5702 SM Helmond (NL); Hack, Cornelis Erik, 1111 ND Diemen (NL); van Engelenburg, Franciscus Antonius Cornelis, 3555 EE Utrecht (NL)
(74) Representative: Prins, Adrianus Willem, Mr. Ir.

(57) **Abstract**

Method for the detection of a pathogenic form of a prion protein in a sample, comprising providing a container, pretreating the container to deposit on a surface of the container a coating of a cellulose derivative capable of favoring the binding of pathogenic prion protein to said container surface over the binding of cellular prion protein, incubating the sample in said container to bind any pathogenic prion protein present in the sample to said container surface, labelling the thus immobilized pathogenic prion protein, if present, with an appropriate labelling agent using an anti-prion antibody capable of binding to pathogenic prion protein and detecting the presence of labelling agent attached to the container surface. Coating a surface of a microtitre plate well with nitrocellulose allows to perform an ELISA for quantification of pathogenic prions in a suspected sample, especially after enzymatic digestion of the sample with an enzyme like proteinase K, by enhancing binding of pathogenic prions and/or reducing binding of cellular (non-pathogenic) prions.

## Description

### FIELD OF THE INVENTION

This invention relates to the field of prion diseases. These diseases, also called transmissible spongiform encephalopathies (TSE), include bovine spongiform encephalopathy (BSE) or mad cow disease in cattle, scrapie in sheep, and Creutzfeldt-Jakob Disease (CJD) in human.

More specifically, the invention relates to the field of diagnostic methods and diagnosis of prion diseases or TSE. Typically, such diagnostic methods seek to detect a (pathogenic) conformer of the natural (non-pathogenic) prion protein, especially in animal or human brain, in lymphoid tissues, or in body fluids in which such conformer may be found, such as blood / plasma / serum or urine.

### BACKGROUND OF THE INVENTION

This invention relates to methods to diagnose prion diseases or TSE in tissue samples and other biological samples (such as body fluids).

Prion diseases or TSE are a group of fatal non-inflammatory neurodegenerative disorders exemplified by BSE in cattle, scrapie in sheep or CJD in man.¹ Prion diseases are infectious diseases, supposedly transmitted by a conformer of the naturally present prion protein (PrP), though cases may also occur spontaneously or from a genetic predisposition. TSE are characterised by a relatively long incubation period (e.g. >10 years in human), and typically cause accumulation of the conformer (the pathogenic or scrapie form of prion protein, PrP^{Sc}) in brain and sometimes also in lymphoid tissues.^{2,3}

The prion protein is an endogenous protein, that also under normal conditions is expressed in brain, and in various other tissues. This PrP^{C} (for normal or cellular prion protein) and the pathogenic or scrapie form PrP^{Sc} are different conformations of the same protein. Furthermore PrP^{C} and PrP^{Sc} differ in their physico-chemical and biochemical properties: PrP^{C} is soluble, exists in a non-aggregated form, and is sensitive to degradation by proteases, whereas PrP^{Sc} is insoluble, exists in an aggregated form, and is relatively resistant to degradation by proteases.⁴ It is hypothesised that a direct protein-protein contact between PrP^{Sc} and PrP^{C} is needed to induce a conformational change of PrP^{C} into PrP^{Sc}. Ultimately PrP^{Sc} forms aggregates and accumulates in the brain. At the final stage of the disease, neurodegenerative changes occur in the brain, associated with severe neurological dysfunction, and ultimately death.

The PrP gene of various animal species has been cloned and sequenced.⁵ The protein is encoded within a single exon, and consists of a long N-terminal signal sequence followed by approximately 250 amino acid (AA) residues. The C-terminus contains a sequence of about 22 residues encoding for linker site for a glycosyl-phosphatidylinositol anchor, a property shared with many other membrane proteins. Furthermore, the protein contains one disulphide bridge and two potential Asparagine-glycosylation sites. The sequence of the protein is well conserved among various animal species.

Though differences in the secondary structure of PrP^{Sc} and that of PrP^{C} have been identified, the structural basis for the enhanced aggregatibility and proteolytic resistance of PrP^{Sc} as compared to those of PrP^{C} is still not resolved. In addition, the molecular basis for the existence of different strains of PrP^{Sc}, i.e. prions with different incubation times, is lacking, though it is assumed that these strains likely represent PrP^{Sc} with subtle differences in conformation.⁶

Crucial for the diagnosis of TSE is the detection of PrP^{Sc} in brain, using e.g. immuno-histochemistry or Western blotting. Detection of PrP^{Sc} in more easily accessible sample, preferably blood, would also add to the diagnosis of TSE. However there is only very incomplete data available suggesting that PrP^{Sc} is present in blood and urine.^{7,8,9,10}

For the *in vitro* diagnostic methods, polyclonal and monoclonal antibodies are usually applied, ^{11,12} but most antibodies against prions described in the literature so far, do not discriminate between PrP^{C} and PrP^{Sc}. There are some papers and patents describing conformer-specific antibodies, but they are not suitable for regular immuno-diagnostics.¹³

To overcome the problem of lack of specificity of the antibodies used, diagnostic methods mostly are based on differences in physico-chemical and biochemical characteristics of PrP^{C} and PrP^{Sc}. A widely used discrimination method is digestion with an enzyme, such as proteinase K (PK), which will, under specific conditions, result in a total digestion of PrP^{C} while PrP^{Sc} remains relatively resistant to the protease treatment. Subsequently the remaining PrP^{Sc} can be detected with classical immunological methods like Western blot, ELISA and similar. Other conformer-discriminating methods are based on differences in solubility, selective precipitation or state of aggregation.

Detection of prion proteins by Western Blot analysis is a laborious and time-consuming method and therefore not very useful. Use of an immunoassay, such as an ELISA, generally is much more practical, but seems to be unsuitable for the specific, sensitive and reliable detection of PrP^{Sc} after PK treatment of a sample. With a sandwich ELISA, a problem is caused by the detergent used in the PK digestion. Said detergent may interfere with the binding of prions to the antibody-coated microtitre plate. Another problem is caused by the sensitivity of the catcher antibody for possible remnant PK activity. A problem with a direct ELISA, in which the prion protein is bound directly to a microtitre plate, is that the PrP^{Sc} happens to bind only weakly, especially after treatment with PK. This makes quantitative detection of PrP^{Sc} virtually impossible. Without enzymatic treatment with PK, another difficulty is that PrP^{C} and PrP^{Sc} bind equally strong to the microtitre plate.

DD 236400 and EP 211229 disclose that the direct binding of antibodies and other proteins to the surface of wells in a microtitre plate can generally be improved by applying a thin nitrocellulose coating onto said surface. They do not relate to prion proteins and do not contain any indication that the application of a nitrocellulose coating may have a different effect on proteins with a different conformation.

An object of this invention is to provide a method for detecting pathogenic forms of a prion protein in a sample which overcomes the above mentioned problems.

A particular object of the invention is to provide a diagnostic method which allows a sensitive and reliable quantification of PrP^{Sc} in a sample.

Another object of the invention is to provide an ELISA-type immunoassay for the quantification of PrP^{Sc} in a sample subjected to an enzymatic digestion with an enzyme such as proteinase K, which allows a strong binding of the PrP^{Sc} to the microtitre plate.

Another object of the invention is to provide an ELISA for quantitatively measuring pathogenic forms of a prion protein wherein a strong binding of PrP^{Sc} to the surface of the wells in a microtitre plate is achieved while allowing direct readout of an enzymatically produced dye in a microplate reader.

Another object of the invention is to provide an immunoassay for reliable and sensitive quantification of pathogenic forms of a prion protein wherein the binding of these pathogenic forms to the microtitre plate is enhanced while the binding of the natural or cellular prion protein is reduced.

Another object of the invention is to provide an improved method for the diagnosis of prion diseases or TSE wherein the risk of false positive results, false negative results, or both, is reduced.

### SUMMARY OF THE INVENTION

The invention achieves the above mentioned objectives by the use of a microtitre plate which has been pretreated in at least some of its wells to favor the binding of pathogenic prion protein over cellular prion protein, by improving the binding thereto of pathogenic forms of prion protein, or reducing the binding thereto of cellular prion protein, or both. This invention uses a microtitre plate with an enhanced binding of the pathogenic or scrapie form of prion protein (PrP^{Sc}) as compared to a normal microtitre plate. Further, this microtitre plate preferably has a reduced binding of the normal or cellular form of prion protein (PrP^{C}) as compared to a normal microtitre plate. This will result in a more specific but also more sensitive, simpler, and quicker detection system for PrP^{Sc} as compared to Western Blot analysis. Together with the high throughput of the microtitre plate format, an ELISA with a chemically modified transparent microtitre plate as disclosed herein facilitates a more suitable diagnostic tool than a Western Blot assay. This invention will be useful for diagnosis of animal and human prion diseases.

The chemical modification of the microtitre plate comprises an application of a clear nitrocellulose layer on the bottom of wells of a regular microtitre plate, or a clear layer of other suitable cellulose derivatives, and thereby results in a more specific and sensitive, but also simpler, quicker, and higher through-put detection system for PrP^{Sc} as compared to Western Blot analysis.

Detection of PrP^{C} and PrP^{Sc} in various biological materials with several antibodies is shown on the chemically modified microtitre plate. The invention will be more fully understood after a consideration of the following description of the invention.

### BRIEF DESCRIPTION OF THE DRAWINGS

### Figure 1

Binding characteristics of PrP^{Sc} and PrP^{C} on a nitrocellulose-coated plate (NC-plate) as compared to a standard microtitre plate (Std-plates). Homogenates (0.1%) of scrapie-infected hamster brain (strain 263K) or normal mouse brain were treated with or without proteinase K (PK). Subsequently the samples were incubated on NC- or Std-plates. Bound prion proteins were detected using an HRP-labelled anti-prion antibody and visualised with TMB substrate. A detailed description of the methodology can be found elsewhere in this patent.

### Figure 2

Analysis of prion proteins in brain samples of cattle (BSE infected and uninfected), hamster (263K-infected and uninfected), sheep (scrapie infected and uninfected), human (sCJD and vCJD infected and uninfected). Homogenates (0.1%) were treated with or without PK. Subsequently the samples were incubated on a NC-plate. Bound prion proteins were detected using an HRP-labelled anti-prion antibody and visualised with TMB substrate. A detailed description of the methodology can be found elsewhere in this patent.

### Figure 3

Detection of bound prion proteins on NC-plates using various anti-prion antibodies. Homogenates (0.1%) of scrapie-infected hamster brain (strain 263K) or normal mouse brain were treated with or without PK. Subsequently the samples were incubated on a nitrocellulose-coated microtitre plate. Bound prion proteins were detected using various anti-prion antibodies (3F4, 6H4, or 1E4) and visualised by successive incubation with HRP-labeled anti-mouse antibody and TMB substrate. A detailed description of the methodology can be found elsewhere in this patent.

### DETAILED DESCRIPTION OF THE INVENTION

Many immunoassays, especially those used for quantification in biological materials, exist in the characteristic sandwich format, in which an antibody is coated to a polystyrene microtitre well. This so-called catching antibody is able to bind a protein specifically. Subsequently the assay is completed by incubation of a second protein-specific antibody that contains an enzymatic or fluorescent label, making quantification of the amount of protein present in the sample possible.

In the case of prion proteins, the majority of prion-specific antibodies reacts both with PrP^{Sc} and PrP^{C} and a digestion with PK is necessary for specific detection of PrP^{Sc}. The digestion with PK of PrP^{Sc} containing samples to degrade PrP^{C} is performed in the presence of a relatively high concentration of detergent for optimal digestion. Such concentrations of detergents however can easily interfere with the binding of prions onto antibody-coated microtitre plates. Complicated optimisation of reaction conditions of such sandwich assays is necessary to reveal conditions for sensitive detection of the residual prion protein after PK digestion. Another drawback of the sandwich ELISA in this methodological approach is the sensitivity of catching antibody for possible remnant PK activity after digestion. After digestion of the sample, a protease inhibitor (PMSF, for example) is added to block the PK activity, however the effectivity of these protease inhibitors is doubtful (non-published internal data).

Therefore we considered using a method of directly binding prion proteins to a microtitre plate. The initial observation that led to the present invention is the finding that PrP^{Sc} present in biological materials like brain homogenates weakly binds to normal microtitre plates especially after treatment with PK. Therefore quantitative detection of PrP^{Sc} directly applied to normal microtitre plates is virtually impossible. Binding of prion to nylon (e.g. PVDF or Biodyne) or nitrocellulose membranes either directly ('spotblot') or by Western Blot techniques is possible, but lacks simple quantification of the amount of prion proteins. Titration methods of various proteins with spotblots making semi-quantification possible have been described, however these methods are very time-consuming and difficult to automate.^{14,15} Also paper-punched disks of cellulose acetate, hydrazinolyzed dacron, or nitrocellulose placed in a normal microtitre plate have been tried in research settings, making quantification possible in a microtitre plate format. Besides the laborious and cumbersome preparation of microtitre plates provided with such disks, direct readout of an enzymatically produced dye in a microtitre plate reader is not possible, due to obstruction of the beam of light by the disks. The produced dye must be transferred into an empty microtitre plate to make quantification possible. Also so-called ELISPOT plates i.e. microtitre plates with nylon or nitrocellulose bottoms, lack transparancy and have similar problems. Such methods are not very practical, especially in high through-put applications, like the screening for BSE in brain of slaughtered cattle.

In this invention, the excellent binding characteristics of nitrocellulose for pathogenic prion proteins are combined with the evident advantages of using a microtitre plate format. Nitrocellulose dissolved in a suitable solvent (non-aggressive towards the plate), such as for example methanol or ethanol, is added to each well of the microtitre plate and subsequently the solvent is evaporated. Under appropriate conditions as specified herein a clear nitrocellulose layer is formed at the bottom of the microtitre plate. After binding of pathogenic prion proteins to the nitrocellulose layer, the assay is completed like a normal ELISA.

The use of a clear nitrocellulose layer to a normal microtitre plate can be very suitable in the field of prion research and diagnostics. The application of this invention to the detection of pathogenic prion proteins in various materials derived from man, cattle, sheep, and rodent prion models is shown in the examples below.

The present invention provides a method for the detection of a pathogenic form of a prion protein in a sample, comprising providing a container, pretreating the container to deposit on a surface of the container a coating of a cellulose derivative capable of favoring the binding of pathogenic prion protein to said container surface over the binding of cellular prion protein, incubating the sample in said container to bind any pathogenic prion protein present in the sample to said container surface, labelling the thus immobilized pathogenic prion protein, if present, with an appropriate labelling agent using an anti-prion antibody capable of binding to pathogenic prion protein and detecting the presence of labelling agent attached to the container surface.

Preferably, the method is an Enzyme-Linked Immuno Sorbent Assay (ELISA) method for the detection of a pathogenic form of a prion protein in a sample, comprising providing a container, pretreating the container to deposit on a surface of the container a coating of a cellulose derivative capable of favoring the binding of pathogenic prion protein to said container surface over the binding of cellular prion protein, incubating the sample in said container to bind any pathogenic prion protein present in the sample to said container surface, labelling the thus immobilized pathogenic prion protein, if present, with an appropriate labelling enzyme using an anti-prion antibody capable of binding to pathogenic prion protein, incubating the container with an appropriate substrate for the labelling enzyme and detecting conversion of the substrate into a coloured, fluorescent or luminescent product.

Therefore, in a preferred embodiment, the method is an enzyme-linked immunosorbent assay (ELISA) using a labelling enzyme as labelling agent, wherein the presence of labelling enzyme attached to the container surface is detected by incubating the container with an appropriate substrate for the labelling enzyme and detecting conversion of the substrate into a coloured, fluorescent or luminescent product.

Preferably, the cellulose derivative used favors the binding of pathogenic prion protein over cellular prion protein by enhancing the binding of pathogenic prion protein to the container surface, or reducing the binding of cellular prion protein to the container surface, or both. Thereby, the risk of false positive or false negative results is significantly reduced and quantitation improved. As will be demonstrated in the examples, a proper pretreatment of the solid phase with a cellulose derivative results in a substantial decrease of the binding of cellular prion protein.

It is preferred that the container is transparent. Usually, the container is a well of a microtitre plate, which may be a conventional or unconventional microtitre plate, such as a conventional microtitre plate made of polystyrene or polyvinylchloride.

The coating of a cellulose derivative which is deposited on a surface of the container preferably is transparent. If the container itself and the coating on a surface thereof are both transparent, direct readout of the result in for example a conventional ELISA reader is possible.

The cellulose derivative used preferably is insoluble in water, to prevent that it dissolves when subsequently the sample is incubated in the container. An appropriate cellulose derivative is a nitrocellulose, but other suitable cellulose derivatives, such as collodium, acetylcellulose, etc., may be used as well.

Preferably, the pretreatment comprises incubating the container with a solution of an appropriate cellulose derivative in a non-aggressive solvent, such as methanol or ethanol, followed by evaporation of the solvent. Aggressive solvents, like acetone, could affect the desired transparancy of the container and are less suitable for that reason. Methanol is the preferred solvent. Evaporation is usually made at room temperature or at somewhat higher temperatures, up to about 50 or 60 °C or even up to about 100 °C. Evaporation may also be carried out under reduced pressure (in vacuo).

It has been found that the solution may contain from 0.001 to 20 mg/ml of the cellulose derivative. Preferably, the concentration of the cellulose derivative is at least 0.01 mg/ml. The solution is used in an amount which secures that the cellulose derivative is deposited onto the surface of the container in an amount of from 1 to 20,000 ng/mm², preferably from 8 to 16,000 ng/mm². When smaller amounts are deposited, the improvement in binding of pathogenic prion protein and the reduction of binding of cellular prion protein are only marginal, and when higher amounts are deposited, the transparancy of the coating becomes sub-optimal. Usually, with conventional microtitre plates, 10 to 250 microliter solution, preferably 25 to 50 microliter, is filled in each well to be coated. The coating will usually have a thickness of from 100 to 1000 nm, preferably 300 to 700 nm.

According to the invention, it is very much preferred that the sample is pretreated with an enzyme capable of digesting cellular prion protein. A suitable enzyme is proteinase K, but others which effectively degrade any cellular prion protein while being much less effective in the degradation of pathogenic prion proteins may be used as well. An example of another useful enzyme is pronase.

If performed properly, such enzymatic pretreatment results in a full digestion of normal (cellular) prion protein, while the effect on pathogenic prion protein is much more restricted. Although the enzymatic treatment will usually remove an N-terminal part of the molecule, a large part (having a molecular weight of about 27-30 kDa) of pathogenic prion protein remains intact. The epitopes of various anti-prion antibodies, such as 1E4, 3F4 and 6H4, are all contained in this proteinase K resistant part of pathogenic prion protein.

In this embodiment of the invention, it is highly advantageous that the pretreatment of the container with a cellulose derivative favors the binding of enzymatically pretreated pathogenic prion protein over enzymatically pretreated cellular prion protein by enhancing the binding of enzymatically pretreated pathogenic prion protein to the container surface, or reducing the binding of enzymatically pretreated cellular prion protein to the container surface, or both. Thereby, the risk of false positive or false negative results is significantly reduced and quantitation improved. As will be demonstrated in the examples, in the absence of such pretreatment with a cellulose derivative, enzymatically pretreated pathogenic prion protein hardly binds to the solid phase. When a properly pretreated solid phase is used, however, enzymatically pretreated pathogenic prion protein strongly binds. In a preferred embodiment of the invention, the enzymatic digestion is stopped by briefly heating the sample to a temperature of from 70 to 100 °C to inactivate the enzyme. Thereafter, the sample is treated with detergent at a temperature of from 70 to 100 °C to denature the protein in the sample.

The labelling of any pathogenic prion protein immobilized onto the container surface is performed either directly with an enzyme-labelled anti-prion antibody, or indirectly with a non-labelled anti-prion antibody followed by an enzyme-labelled antibody capable of binding to the anti-prion antibody. The person skilled in the art is well aware of these, and other alternatives. A very practical approach is the indirect method, because the enzyme-labelled antibody (such as enzyme-labelled goat-antimouse antibody) is a generally useful reagent which can be used in many different assays.

The anti-prion antibody preferably is a monoclonal antibody. Antibodies binding to an epitope in the protease K resistant part of pathogenic prion protein are preferred. Suitable examples are the monoclonal antibodies 3F4, 6H4 and 1E4. Use of monoclonal antibody 1E4 (CNCM 1-2906) is specially preferred.

Any conventional or unconventional labelling enzyme may be used. To mention a well known example, a peroxidase, such as Horse Radish Peroxidase (HRP), may be used as a labelling enzyme. Other enzymes, such as alkaline phosphatase, etc., may be used instead.

The substrate to be used depends on the labelling enzyme chosen. In the case of HRP, it is possible to use 3,3',5,5'-tetramethylbenzidine as a substrate for the labelling enzyme. The person skilled in the art knows many alternatives for the labelling enzyme and the substrate.

It is however much preferred that the enzyme and substrate are chosen such as to allow easy and preferably quantitative readout of the result, i.e. that they are chosen such that the substrate conversion results in a coloured material which is detected by direct readout of the absorbance.

Although the invention includes methods merely aiming at a qualitative detection of a pathogenic form of a prion protein in a sample, it is preferred that the quantitative occurrence of a pathogenic form of a prion protein in a sample is determined.

In a practical embodiment of the invention, the ELISA includes a parallel ELISA without pretreatment of the container as a control of complete enzymatic digestion of cellular prion protein.

In the method of the invention, the sample will normally be derived from brain or lymphoid tissue of a human or animal. Other kinds of samples may be useful as well, in particular body fluids such as blood, plasma, cerebrospinal fluid, saliva, sputum, seminal fluid, vaginal fluid and urine. Post-mortem analysis allows to diagnose the cause of death and/or (with animals) the infection status of the animal. Analysis of live individuals may be useful with an eye on treatment and/or measures to prevent further infection.

This invention further provides a method for separating pathogenic prion protein from a mixture which contains pathogenic prion protein and cellular prion protein comprising contacting the mixture with a surface which preferentially binds pathogenic prion protein and separating the non-bound material from said surface.

Herein, it is preferred that the surface is made of, or coated with, a water-insoluble cellulose derivative, such as nitrocellulose. Said surface may be a surface of a microtitre plate well coated with a layer of nitrocellulose. However, it may also be a surface of a bead or column filling which is made of, or coated with, nitrocellulose.

Also in this aspect of the invention, it is preferred that the mixture is pretreated with an enzyme capable of digesting cellular prion protein, such as proteinase K. Again, it is preferred that after the enzymatic digestion first the digestion enzyme is inactivated and then the protein which is present in the mixture is denatured.

The invention will now be illustrated by the following examples, which by no means intend to restrict the invention.

### EXAMPLES

To demonstrate the effect of a clear nitrocellulose layer on the prion binding characteristics of a microtitre plate, we performed a direct ELISA using a nitrocellulose-coated microtitre plate (NC-plate) or a standard microtitre plate (Std-plate). Homogenates (0.1%) of scrapie-infected hamster brain (strain 263K) or normal mouse brain were treated with or without PK and incubated on the NC-plate - nitrocellulose coating was 0.1% - or Std-plate. The bound prion proteins were detected using the anti-prion antibody 1E4 labelled with Horse Radish Peroxidase (HRP) and subsequently stained with 3,3',5,5'-tetramethylbenzidine (TMB) substrate.

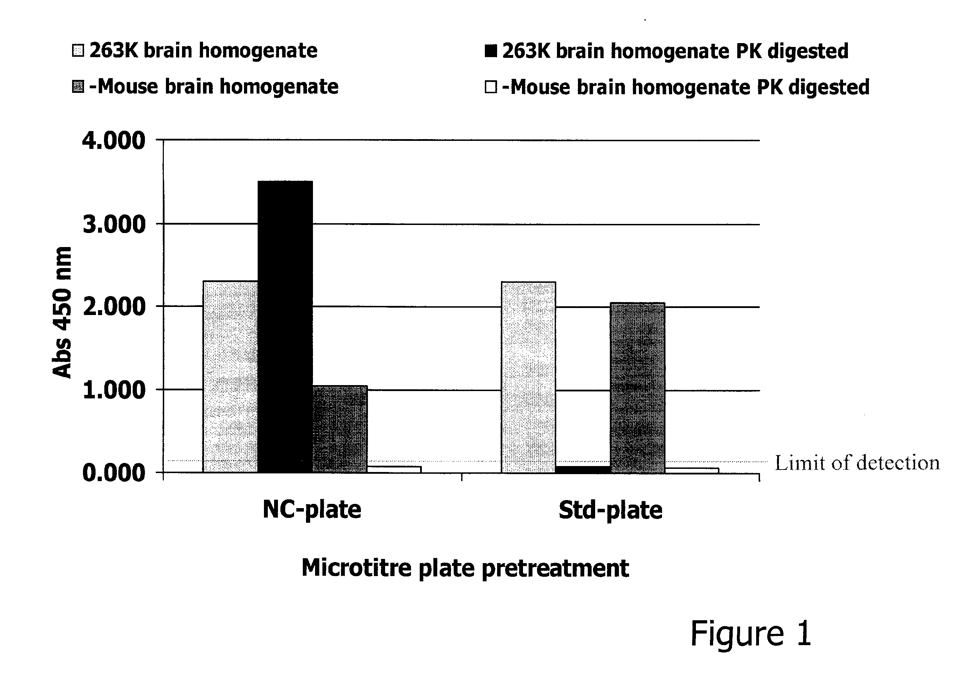

As shown in Figure 1, the prion protein present in PK-digested scrapie-infected hamster brain (PrP^{Sc}) binds to the NC-plate, but not to the Std-plate. Furthermore the prion protein present in undigested normal mouse brain (PrP^{C}) binds worse to the NC-plate than to the Std-plate.

To demonstrate the prion binding characteristics of a NC-plate, we performed a direct ELISA using brain samples derived from cattle (BSE infected and uninfected), hamster (263K-infected and uninfected), sheep (scrapie infected and uninfected), human (sCJD and vCJD infected and uninfected). These samples were treated with of without PK and applied to a NC-plate with a 0.1% nitrocellulose coating. The bound prion proteins were detected using the anti-prion antibody 1E4 labelled with HRP and subsequently stained with TMB substrate.

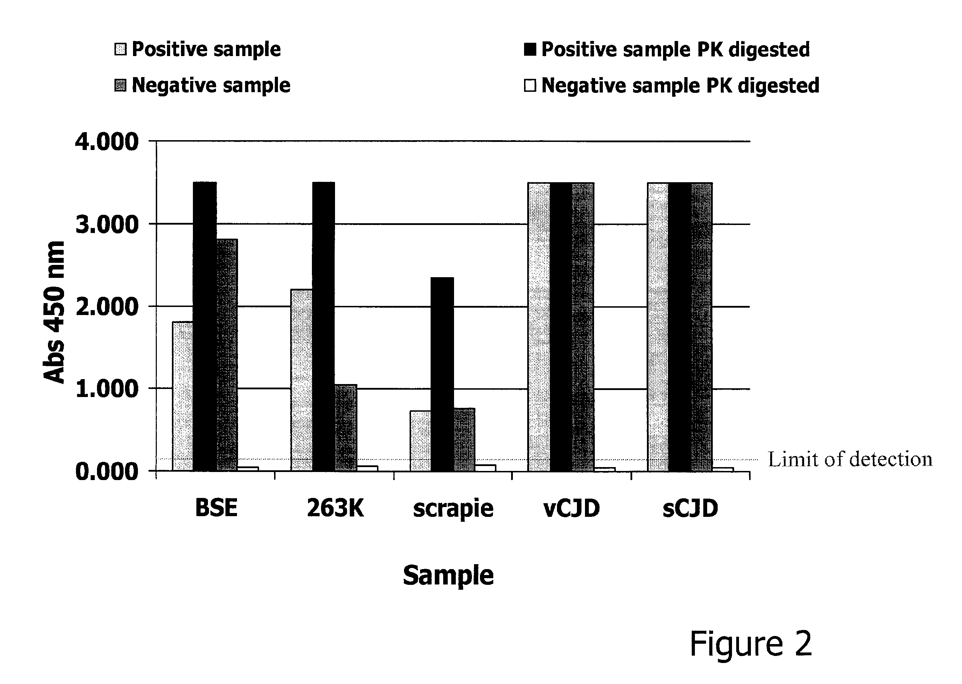

The PK-digested samples from TSE-infected animal or patients are found strongly positive, whereas the PK-digested samples from uninfected animal and human are found negative (Figure 2). All undigested samples are found positive demonstrating the pivotal dependance of the assay on PK digestion for specific detection of PrP^{Sc}.

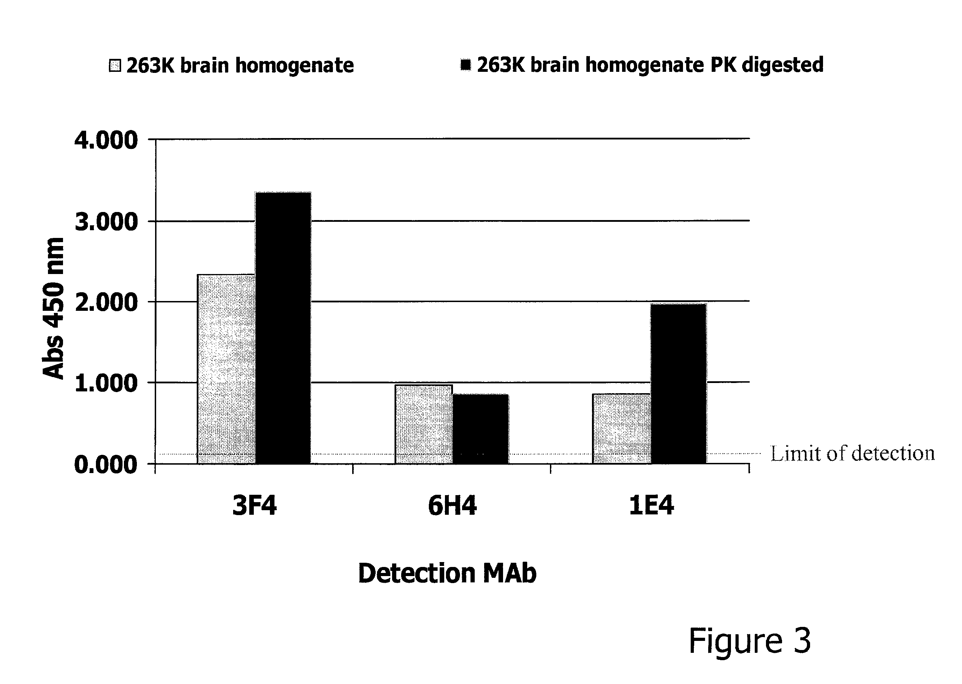

To demonstrate the suitability of NC-plates for the detection of prion proteins, we performed a direct ELISA using various anti-prion antibodies (Figure 3). Homogenates (0.1%) of scrapie-infected hamster brain (strain 263K) or normal mouse brain were treated with or without PK. Subsequently the samples were incubated on a NC-plate. Bound prion proteins were detected using various anti-prion antibodies (3F4 [DAKO], 6H4 [Prionics], or 1E4 [Sanquin]) and visualised by successive incubation with HRP-labeled anti-mouse antibody and TMB substrate.

All antibodies tested positive for the PK-digested scrapie-infected hamster brain sample (PrP^{Sc}).

### METHODS

Nitrocellulose was dissolved in methanol or ethanol to a final concentration of 0.01 or 20 mg/ml. A volume of 40 µl of this mixture was added into each well of a 96-well microtitre plate (NUNC maxisorp type F96) and the solvent was evaporated by heating the plate in an oven for 30-60 minutes at 80°C resulting in a clear nitrocellulose layer remaining at the bottom of the microtitre plate. The amount of nitrocellulose applied corresponds to 8 - 16,000 ng/mm².

Brain samples of various species were homogenized in phosphate buffered saline - pH 7.4 (PBS) or 0.25 M sucrose solution to a final concentration of 10% (w/v). These homogenates were further diluted to a final concentration of 0.1 % with a digestion buffer (100 mM Tris/HCl pH 7.5, 0.05% SDS) and incubated for 30 min at 50 °C with or without PK (final concentration 30 µg/ml ~ 0.6 U/ml). To stop the PK digestion the samples were heated to 96°C for 1 min and then cooled to room temperature (RT). After treatment the volume was increased by one-third by addition of sample buffer (200 mM TRIS/HCl [pH 8.5], 6% SDS) and the mixture was incubated for 10 min at 96°C.

Subsequently 40 µl of treated sample was transferred to the wells of the nitrocellulose-coated microtitre plate and incubated for 1 hour at RT, while shaken. After washing with washing buffer (PBS, 0.15% Tween-20), the plate was incubated for 1 h at RT with 40 µl of anti-prion antibody diluted in incubation buffer (PBS, 0.15 % Tween-20, 0.5 % PEG 4000). Subsequently the plate was washed and incubated for 1 h with an HRP-labelled goat-anti-mouse antibody. Bound peroxidase was detected by 100 µl/well 3,3',5,5'-tetramethylbenzidine (TMB)/H₂O₂ in 100 mM Acetate buffer (pH 5.5). After incubation for 20 min, the enzyme reaction was stopped by addition of 100 µl of 1 M H₂SO₄ and absorbances were read at 450 nm in an Anthos ELISA reader (690 nm was used as a reference).

### REFERENCES

1. Prusiner SB, Prions (1998). PNAS 95, 13363-13383.
2. Wadsforth JDF et al., Tissue distribution of protease resistant prion protein in variant Creutzfeldt-Jakob disease using a highly sensitive immunoblotting assay (2001). Lancet 358, 171-180.
3. Schreuder BE et al., Preclinical test for prion diseases (1996). Nature 381, 563.
4. Naslavsky N, et al., Characterization of detergent-insoluble complexes containing the cellular prion protein and its scrapie isoform (1997). JBC 272, 6324-6331.
5. Wiessmann C, The ninth Datta Lecture. Molecular biology of transmissible spongiform encepholpathies (1996). FEBS Letters 389, 3-11.
6. Safar J et al., Eight prions strains have PrP^{SC} molecules with different conformations (1998). Nature Medicine 4, 1157-1165.
7. Schmerr MJ and Jenny A, A diagnostic test for scrapie-infected sheep using a capillary electrophoresis immunoassay with fluorescent-labeled peptides (1998). Electrophoresis 19, 409-413.
8. Houston F et al.Transmission of BSE by blood transfusion in sheep (2000). Lancet 356, 999-1000.
9. Houston F et al. Transmission of prion diseases by blood transfusion (2002). J Gen Virol. 83, 2897-905
10. Shaked GM et al., A protease-resistant protein isoform is present in urine of animals and humans affected with prion diseases (2001). J Biol Chem 276 (34), 1479-82.
11. Kascsak RJ et al., Mouse polyclonal and monoclonal antibody to scrapie-associated fibrin proteins (1987). J Virol 61, 3688-3693.
12. Laffling AJ, et al. A monoclonal antibody that enables specific immuno-histological detection of prion protein in bovine spongiform encephalopathy cases (2001). Neurosci Lett 300, 99-102.
13. Korth C et al., Prion (PrP^{Sc})-specific epitope defined by a monoclonal antibody (1997). Nature 390, 74-77.
14. Dattamajumbar AK et al., Rapid cloning of any rearranged mouse immunoglobulin variable genes (1996). Immunogenetics 43, 141-151.
15. Palfree GE and Elliott BE, An enzyme-linked immunosorbent assay (ELISA) for detergent solubilized Ia glycoproteins using nitrocellulose membrane disks (1982). J Immunol Meth 52, 395-408.

## Claims

1. A method for the detection of a pathogenic form of a prion protein in a sample, comprising providing a container, pretreating the container to deposit on a surface of the container a coating of a cellulose derivative capable of favoring the binding of pathogenic prion protein to said container surface over the binding of cellular prion protein, incubating the sample in said container to bind any pathogenic prion protein present in the sample to said container surface, labelling the thus immobilized pathogenic prion protein, if present, with an appropriate labelling agent using an anti-prion antibody capable of binding to pathogenic prion protein and detecting the presence of labelling agent attached to the container surface.

2. A method according to claim 1, which is an enzyme-linked immunosorbent assay (ELISA) using a labelling enzyme as labelling agent, wherein the presence of labelling enzyme attached to the container surface is detected by incubating the container with an appropriate substrate for the labelling enzyme and detecting conversion of the substrate into a coloured, fluorescent or luminescent product.

3. A method according to claim 1 or 2, wherein the cellulose derivative used favors the binding of pathogenic prion protein over cellular prion protein by enhancing the binding of pathogenic prion protein to the container surface, or reducing the binding of cellular prion protein to the container surface, or both.

4. A method according to any one of claims 1-3, wherein the container is transparent.

5. A method according to any one of claims 1-4, wherein the container is a well of a microtitre plate.

6. A method according to any one of claims 1-5, wherein the coating of a cellulose derivative deposited on a surface of the container is transparent.

7. A method according to any one of claims 1-6, wherein the cellulose derivative used is insoluble in water.

8. A method according to claim 7, wherein the cellulose derivative is a nitrocellulose.

9. A method according to any one of claims 1-8, wherein said pretreatment comprises incubating the container with a solution of an appropriate cellulose derivative in a non-aggressive solvent, such as methanol or ethanol, followed by evaporation of the solvent.

10. A method according to claim 9, wherein the solution contains from 0.001 to 20 mg/ml of the cellulose derivative.

11. A method according to any one of claims 1-10, wherein the cellulose derivative is deposited onto the surface of the container in an amount of from 1 to 20,000 ng/mm².

12. A method according to any one of claims 1-11, wherein the sample is pretreated with an enzyme capable of digesting cellular prion protein.

13. A method according to claim 12, wherein the enzyme is proteinase K.

14. A method according to claim 12 or 13, wherein the enzymatic digestion is stopped by briefly heating the sample to a temperature of from 70 to 100°C to inactivate the enzyme.

15. A method according to claim 14, wherein the sample after the inactivation of the enzyme is treated with detergent at a temperature of from 70 to 100 °C to denature the protein in the sample.

16. A method according to any one of claims 12-15, wherein the pretreatment of the container with a cellulose derivative favors the binding of enzymatically pretreated pathogenic prion protein over enzymatically pretreated cellular prion protein by enhancing the binding of enzymatically pretreated pathogenic prion protein to the container surface, or reducing the binding of enzymatically pretreated cellular prion protein to the container surface, or both.

17. A method according to any one of claims 1-16, wherein the labelling of any pathogenic prion protein immobilized onto the container surface is performed either directly with an enzyme-labelled anti-prion antibody, or indirectly with a non-labelled anti-prion antibody followed by an enzyme-labelled antibody capable of binding to the anti-prion antibody.

18. A method according to any one of claims 1-17, wherein a peroxidase, such as Horse Radish Peroxidase, is used as a labelling enzyme.

19. A method according to any one of claims 1-18, wherein 3,3',5,5'-tetramethylbenzidine is used as a substrate for the labelling enzyme.

20. A method according to any one of claims 1-19, wherein the substrate conversion results in a coloured material which is detected by direct readout of the absorbance.

21. A method according to any one of claims 1-20, wherein the quantitative occurrence of a pathogenic form of a prion protein in a sample is determined.

22. A method according to any one of claims 1-21, wherein the ELISA includes a parallel ELISA without pretreatment of the container as a control of complete enzymatic digestion of cellular prion protein.

23. A method according to any one of claims 1-22, wherein the sample is derived from brain or lymphoid tissue of a human or animal.

24. A method according to any one of claims 1-22, wherein the sample is a body fluid, such as blood, plasma, cerebrospinal fluid, saliva, sputum, seminal fluid, vaginal fluid or urine.

25. A method for separating pathogenic prion protein from a mixture which contains pathogenic prion protein and cellular prion protein comprising contacting the mixture with a surface which preferentially binds pathogenic prion protein and separating the non-bound material from said surface.

26. A method according to claim 25, wherein said surface is made of, or coated with, a water-insoluble cellulose derivative, such as nitrocellulose.

27. A method according to claim 25 or 26, wherein said surface is a surface of a microtitre plate well coated with a layer of nitrocellulose.

28. A method according to claim 25 or 26, wherein said surface is a surface of a bead or column filling which is made of, or coated with, nitrocellulose.

29. A method according to any one of claims 25-28, wherein said mixture is pretreated with an enzyme capable of digesting cellular prion protein, such as proteinase K.

30. A method according to claim 29, wherein after the enzymatic digestion first the digestion enzyme is inactivated and then the protein which is present in the mixture is denatured.
